# EUROPÄISCHE PATENTSCHRIFT

(11) **EP 0 993 418 B1**
(45) Veröffentlichungstag und Bekanntmachung des Hinweises auf die Patenterteilung: **05.09.2001**
(21) Anmeldenummer: 98939546.2
(22) Anmeldetag: 27.06.1998
(51) Int. Cl.: B67C 7/00, B67B 3/06

(54) **VERFAHREN UND ANLAGE ZUM VERSCHLIESSEN VON FLASCHEN MIT STERILEN VERSCHLUSSKAPPEN**
METHOD AND FACILITY FOR SEALING BOTTLES WITH STERILE SEALING CAPS
PROCEDE ET DISPOSITIF DE FERMETURE DE BOUTEILLES PAR DES BOUCHONS STERILES

(30) Priorität: 01.07.1997 DE 19727942
(43) Veröffentlichungstag der Anmeldung: 19.04.2000
(73) Patentinhaber: GEA Finnah GmbH, 48683 Ahaus (DE)
(72) Erfinder: SCHRÖDER, Klaus, D-48683 Ahaus (DE)
(74) Vertreter: Busse, Dietrich, Dipl.-Ing.
(86) Internationale Anmeldenummer: EP9803940
(87) Internationale Veröffentlichungsnummer: WO9901374

(56) Entgegenhaltungen:
- EP-A- 0 447 759
- DE-A- 1 910 548
- DE-A- 2 432 276
- DE-A- 3 009 202
- GB-A- 2 271 347
- US-A- 3 905 317
- US-A- 4 296 769

## Beschreibung

Die Erfindung betrifft eine Maschine zum Verschließen von Flaschen mit Verschlußkappen in einer Ausbildung gemäß dem Oberbegriff des Anspruchs 1. Maschinen dieser Art finden sowohl für Kunststoff- als auch für Glasflaschen in Flaschenabfüllanlagen Anwendung, wobei als Verschlußkappen Schraubkappen, Kronkorken oder sonstige Verschlüsse zum Einsatz kommen können.

Die Erfindung befaßt sich mit dem Problem des Verschließens von Flaschen mit sterilisierten Verschlußkappen und löst dieses Problem durch eine Maschine mit den Merkmalen des Anspruches 1 bzw. durch ein Verfahren mit den Merkmalen des Anspruches 21. Hinsichtlich weiterer Ausgestaltungen wird auf die Ansprüche 2 bis 20 verwiesen.

Verfahren und Maschine nach der Erfindung gewährleisten, daß eine Sterilisierung der Verschlußkappen vorgenommen wird, bevor diese auf die Flaschen aufgesetzt werden, wobei die Aufrechterhaltung der Sterilität bis zum Aufsetzen dadurch gesichert ist, daß die Übergabevorrichtung vollständig in einer Sterilkammer angeordnet ist. Die Trocknungsvorrichtung in der Sterilkammer vermeidet eine Kontaminierung der Flaschenmündungen durch Reste von Sterilisationsmitteln. Dabei ist ein Betrieb mit geringen Taktzeiten möglich, wie das in Hochleistungs-Abfüllanlagen erforderlich ist.

Weitere Einzelheiten und Wirkungen ergeben sich aus der nachfolgenden Beschreibung eines in der Zeichnung dargestellten Ausführungsbeispiels des Gegenstandes der Erfindung. In der Zeichnung zeigen:
- Fig. 1: eine erfindungsgemäße Maschine in einer teilweise aufgeschnittenen Seitenansicht,
- Fig. 2: in einer Seitenansicht die Anordnung der Maschine nach Fig. 1 in einer Anlage zur sterilen Befüllung von Flaschen, und
- Fig. 3: eine schematische Draufsicht auf den Flaschenförderteil der Anlage nach Fig. 2.

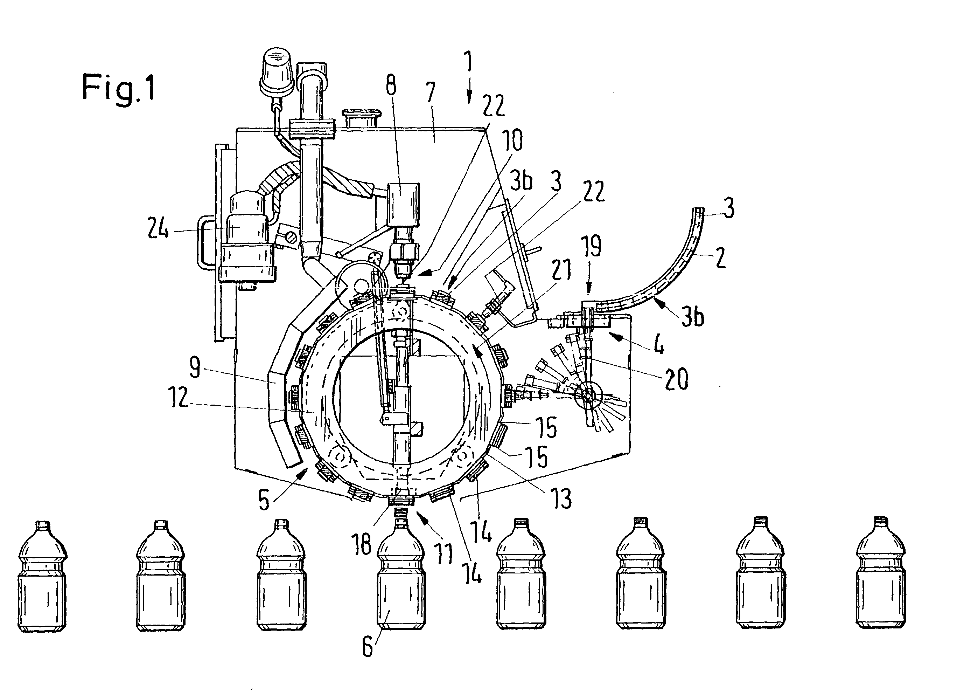

Wie aus Fig. 1 ersichtlich, weist die Maschine 1 eine aus einem nicht dargestellten Vorratsbehälter beschickte Transportbahn 2 zur Zuführung von Verschlußkappen 3 auf, an die sich eine Vereinzelungsvorrichtung 4 anschließt, die die Verschlußkappen 3 in vereinzelter Stellung bereitstellt. Sie umfaßt ferner eine Übergabevorrichtung 5, mittels der die vereinzelten Verschlußkappen 3 auf Flaschen 6 aufgesetzt werden, die entlang einer Flaschentransportbahn unterhalb der Maschine 1 transportiert werden.

In einer Sterilkammer 7 ist eine der Transportbahn 2 an ihrem Ende zugeordnete Vereinzelungsvorrichtung 4 für die Verschlußkappen 3 angeordnet, in der sich im Ausführungsbeispiel auch eine nachgeordnete Übergabevorrichtung 5 befindet. Die an die Übergabevorrichtung 5 übergehenden Verschlußkappen 3 sind damit nicht mehr der Außenluft ausgesetzt, sondern befinden sich nach Verlassen der Transportbahn 2 in steriler Umgebung. Eine der Übergabevorrichtung in deren oberen Kulminationspunkt oberseitig zugeordnete Sprühvorrichtung 8 beaufschlagt die in der Übergabevorrichtung 5 befindlichen Verschlußkappen 3 mit einem Sterilisationsmittel, das in den Innenraum 3b der Verschlußkappen 3 eingesprüht wird. Hierzu befinden sich die Verschlußkappen 3 während des Einspritzens des Sterilisationsmittels in einer Stellung, in der ihr Innenraum 3b nach oben weist. Vor Übergabe der Verschlußkappen 3 auf die Flaschenmündungen müssen die Verschlußkappen 3 daher gewendet werden, so daß der Innenraum 3b nach unten weist und die Verschlußkappen auf die Flaschen 6 aufgesetzt werden können.

Ferner ist der Übergabevorrichtung 5 eine Trocknungsvorrichtung 9 zugeordnet, die im Bereich zwischen der Beaufschlagung von Verschlußkappen 3 mit Sterilisationsmittel und ihrer Übergabe an Flaschen 6 wirksam ist. Die Trocknungsvorrichtung 9 beaufschlagt die Verschlußkappen 3, insbesondere deren Innenräume 3b, mit sterilisierter, ca. 70° heißer Luft, die während des Wendens der Verschlußkappen 3 aus der Sterilisationsstellung 10 in eine Aufsetzstellung 11 Reste von Sterilisationsmittel aus den Verschlußkappen 3 ausbläst.

Im Ausführungsbeispiel ist die Übergabevorrichtung 5 zugleich als Vorrichtung zum Wenden der Verschlußkappen 3 aus der Sterilisationsstellung 10 in die Aufsetzstellung 11 ausgebildet und dabei als Sternrad 12 ausgeführt, das in einer vertikalen Ebene umläuft und über seinen Außenumfang 13 mit Aufnahmegliedern 14 für Verschlußkappen 3 versehen ist, die in regelmäßigen Abständen über dessen Außenumfang 13 verteilt sind. Diese Aufnahmeglieder 14 sind als die Verschlußkappen 3 haltende und diese in ihrem oberen, der Öffnung des Innenraums 3b abgewandten Bereich umgreifende Klemmglieder ausgebildet, die aber auch die gesamten Seitenwandungen der Verschlußkappen 3 umgreifen können. Die Klemmwirkung kann dabei durch Klemmbacken 15 hervorgerufen werden, die z. B. mit Elastomergliedern versehen sind, die die Verschlußkappen 3 haltend aufnehmen. Die Verschlußkappen 3 können aus dieser Haltestellung in den Aufnahmegliedern 14 mittels eines Stempels 16 herausbewegt werden, der entlang einer vertikalen Achse 17 beweglich ist und auf die in Aufsetzstellung 11 befindlichen Verschlußkappen 3 derart einwirkt, daß der Stempel 16 die Verschlußkappen 3 aus den Aufnahmegliedern 14 herausdrückt und auf die darunter befindliche Flasche 6 aufsetzt.

Um bei dieser Aufsetzbewegung einen freien Fall zu verhindern, umfaßt der Stempel 16 in seinem vorderen Bereich 18 ein Saug- oder Greiforgan, das die jeweilige Verschlußkappe 3 lösbar mit dem Stempel 16 verbindet, bevor diese aus den Aufnahmegliedern 14 ausgestoßen wird.

Zum Aufsetzen einer Verschlußkappe 3 auf eine Flasche 6 wird durch den Stempel 16 ein zusätzlicher, abwärts gerichteter Druck auf die Verschlußkappe 3 ausgeübt, so daß die Verschlußkappe 3 bereits eine gewisse Sicherung auf der Flaschenmündung erhält. Das ist insbesondere bei Schraubkappen wünschenswert, damit diese mit ihrem Sicherungsring bereits das Gewinde übergreifen und durch eine der Maschine 1 nachfolgende Drehvorrichtung endgültig festgelegt werden können.

Alternativ kann der Stempel 16 auch zumindest mit seinem vorderen Teil 18 um die vertikale Achse 17 drehbar sein, um ein Verschrauben der Verschlußkappe bereits während des Aufsetzvorganges zu ermöglichen. Bei kronkorkenähnlichen Verschlußkappen kann auch eine die Seiten des Kronkorkens um einen Mündungswulst der Flasche herumliegende Preßeinheit an dem vorderem Bereich 18 des Stempels 16 angeordnet sein.

Zur Zuführung der Verschlußkappen 3 in die Sterilkammer 7 sieht das dargestellte Ausführungsbeispiel als Transportbahn eine Rutsche 2 vor, die an einer Einlaßöffnung 19 in die Sterilkammer 7 mündet und die Verschlußkappen 3 in einzelner Hintereinanderlage führt und mit nach unten weisendem Innenraum 3b an der Einlaßöffnung 19 bereitstellt. An der Einlaßöffnung 19 werden die Verschlußkappen 3 von der Vereinzelungsvorrichtung 4 übernommen, die beispielsweise die Verschlußkappen 3 mittels eines Stempels 20, z. B. eines Saugstempels, annimmt. Der Stempel 20 ist im Ausführungsbeispiel so ausgebildet, daß er neben einer vertikalen Hubbewegung, die einen Durchgriff durch die Einlaßöffnung 19 ermöglicht, eine überlagerte Schwenkbewegung um eine horizontale Achse ausführen kann und nach einem Schwenkwinkel von etwa 90° in eine horizontale Lage gerät, in der er in einer horizontalen Hubbewegung die aufgenommene Verschlußkappe 3 an das Sternrad 12 der Übergabevorrichtung 5 übergibt, bei der der Stempel 20 die jeweilige Verschlußkappe 3 in ein taktgerecht bereitstehendes Aufnahmeglied 14 des Sternrades 12 drückt.

Der Stempel 20 umfaßt für seine geradlinigen und schwenkenden Bewegungen über eigene geeignete, nicht näher veranschaulichte Antriebe, z. B. einen Elektromagnetantrieb für die Linearbewegung und einen Druckmittelantrieb für ein Schwenkgetriebe.

Die Halterung der Verschlußkappe 3 auf dem Stempel 20 kann beispielsweise mittels Unterdruck oder mittels eines mechanischen Greifers bewirkt werden.

Der Stempel 20 ist mit dem getakteten Umlauf des Sternrades 12 so synchronisiert:, daß jedes Aufnahmeglied 14 beim Passieren der Vereinzelungsvorrichtung 4 mit einer Verschlußkappe 3 bestückt wird.

Das Sternrad 12 transportiert die aufgenommenen Verschlußkappen 3 in Richtung des Pfeiles 21 zunächst an einer Detektoreinheit 22 vorbei, die beispielsweise mittels Photodioden prüft, ob jedes passierende Aufnahmeglied 14 mit einer Verschlußkappe 3 versehen ist.

Im weiteren Bewegungsverlauf werden die Verschlußkappen 3 nacheinander in die Sterilisationsstellung 10 gebracht, in der ihr nach oben weisender Innenraum 3b unterhalb der Sprühvorrichtung 8 gelegen ist und mit Sterilisationsmittel beaufschlagt wird.

Die Sprühvorrichtung 8 kann als Ultraschallzerstäuber für eine Feinzerstäubung des Sterilisationsmittels ausgebildet sein, der über eine vordere Düse 22 das fein zerstäubte Sterilisationsmittel in die Kappeninnenräume 3b einbringt.

Hierzu steht die Sprühvorrichtung 8 mit einem Sterilisationsmitteltank 23 sowie einem Sterillufttank 24 in Verbindung, um so ein Gemisch aus sterilisierter Luft und einem Sterilisationsmittel, beispielsweise Wasserstoffperoxid (H₂O₂), in die Kappeninnenräume 3b einbringen zu können. Eine solche Sprühvorrichtung 8 ist grundsätzlich aus der EP 0 272 538 B1 bekannt. Statt Wasserstoffperoxid können auch andere Sterilisationsmittel, z. B. Heißdampf, Verwendung finden, deren Einsatz sich nach geltenden lebensmittelrechtlichen Vorschriften und den jeweiligen Gegebenheiten richten wird.

Nach der Besprühung der Verschlußkappen 3 mit Sterilisationsmittel werden diese im weiteren Verlauf der Sternradbewegung gewendet, wobei die Trocknungsvorrichtung 9 über den wesentlichen Teil der Bewegung von der Sterilisationsstellung 10 in die Aufsetzstellung 11 wirksam ist und in ihrem Wirkungsbereich die Verschlußkappen 3 mit sterilisierter Luft beaufschlagt. Dadurch wird sichergestellt, daß keine Reste des Sterilisationsmittels in den Kappeninnenräumen 3b verbleiben und eventuell in die Flaschenmündung gelangen können.

Durch die Trocknungsluftbeaufschlagung werden anfangs auch die Außenseiten der Verschlußkappen 3 mit dem Sterilisationsmittel benetzt, das nach Austreiben aus dem Innenraum 3b von der Luft außen an den Seitenwandungen entlanggeblasen wird. Infolge der Erstreckung der Trocknungsvorrichtung 9 längs eines erheblichen Bahnstücks der Umlaufbahn der Übergabevorrichtung 5 können gleichzeitig mehrere Verschlußkappen 3 mit Sterilluft beaufschlagt werden.

Die Sterilkammer 7 steht unter einem Überdruck, so daß durch die Eintrittsöffnung 19 bzw. durch die im Bereich der Aufsetzstellung 11 nach unten hin offene Kammer keine Umgebungsluft von außen eindringen kann, die eine Kontaminierung der Verschlußkappen 3 hervorrufen könnte. Da auch die offenen, bereits gefüllten Flaschen 6 bis zur Aufsetzstellung 11 ebenfalls in Sterilluftumgebung geführt sind, sind auch die Flaschenmündungen steril, so daß insgesamt eine Sterilabfüllung gewährleistet ist. Wenn sich der die Flaschen 6 zur Maschine 1 transportierende Förderer ebenfalls in einem Sterilraum bewegt, ist zweckmäßig der Druck in diesem Sterilraum höher als in der Sterilkammer 7 der Maschine 1, um einen Übertritt von Sterilisationsmittelresten zu den Flaschen 6 hin zu vermeiden. In diesem Fall ist für eine Luftabsaugung aus der Sterilkammer 7 in deren oberen Bereich ein zu einer Pumpe führender Absaugstutzen 24 vorgesehen.

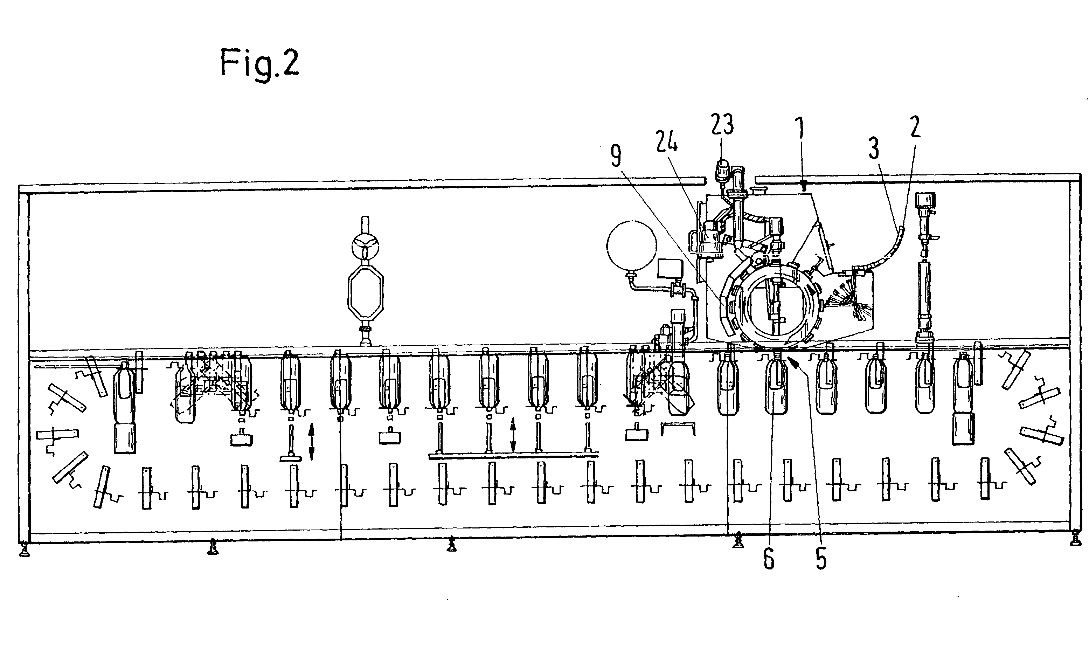

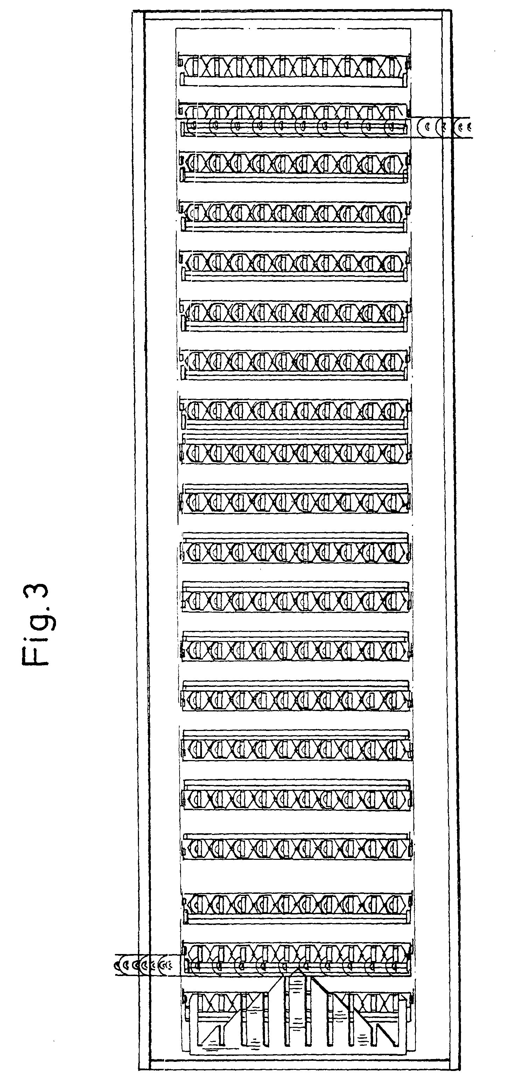

Wenn wie in Fig. 2 und 3 dargestellt die Maschine 1 mit einer mehrreihigen Abfüllanlage zusammenarbeitet, wie sie im Detail in der deutschen Patentanmeldung 197 00 156.4-23 beschrieben ist, kann die Maschine 1 eine entsprechende Parallelanordnung gleichartiger Bauteile zur Zuführung, Bereitstellung und zum Aufsetzen von Verschlußkappen 3 umfassen, um die Flaschen 6 auf mehreren parallellaufenden Transportbändern mit Verschlußkappen gleichzeitig zu verschließen.

Insgesamt ist daher durch die Erfindung ein Verfahren ermöglicht, bei dem Verschlußkappen über eine Transportbahn, in der sie noch frei zugänglich und einer Qualitätskontrolle, etwa im Sinne eines Aussortierens defekter Verschlußkappen, unterworfen sein können, einer Übergabevorrichtung zugeführt werden, die dann die Verschlußkappen in vereinzelter Stellung übernimmt, wobei nach Aussortierung der defekten Verschlußkappen eine hohe Funktionssicherheit der Übergabe gegeben ist. Die Verschlußkappen werden der Übergabevorrichtung mittelbar oder unmittelbar mit nach unten weisendem Innenraum zugeführt, dann von der Übergabevorrichtung so weit gewendet, daß der Innenraum nach oben weist und mit Sterilisationsmittel ausgesprüht werden kann, und danach bei einem weiteren Wenden getrocknet, bevor sie mit nach unten weisendem Innenraum auf die Flaschen aufgesetzt werden.

## Patentansprüche

1. Maschine (1) zum Verschließen von Flaschen (6) mit Verschlußkappen (3), mit einer Transportbahn (2) zur Zuführung der Verschlußkappen (3), einer Vereinzelungsvorrichtung (4) zur Bereitstellung von vereinzelten Verschlußkappen (3) und einer Übergabevorrichtung (5) zum Aufsetzen der vereinzelten Verschlußkappen (3) auf die Flaschen (6), **dadurch gekennzeichnet,** daß zumindest die Übergabevorrichtung (5) in einer Sterilkammer (7) angeordnet und der Übergabevorrichtung (5) eine Sprühvorrichtung (8) zum Einbringen eines Sterilisationsmittels in den Verschlußkappeninnenraum (3b) und eine Trocknungsvorrichtung (9) zum Austreiben von Sterilisationsmittel aus den Verschlußkappen (3) zugeordnet ist.

2. Maschine nach Anspruch 1, **dadurch gekennzeichnet,** daß die Übergabevorrichtung (5) die Verschlußkappen (3) in eine Sterilisationsstellung (10) mit nach oben weisendem Innenraum (3b), in der die Sprühvorrichtung (8) diese mit Sterilisationsmittel beaufschlagt, und aus dieser in eine Aufsetzlage (11) mit nach unten weisendem Innenraum (3b) überführt.

3. Maschine nach einem der Ansprüche 1 oder 2, **dadurch gekennzeichnet,** daß die Übergabevorrichtung (5) ein in vertikaler Ebene umlaufendes Sternrad (12) umfaßt, das mit regelmäßig über seinen Außenumfang (13) verteilten Aufnahmegliedern (14) für die vereinzelten Verschlußkappen (3) versehen ist.

4. Maschine nach einem der Ansprüche 1 bis 3, **dadurch gekennzeichnet,** daß die Sterilkammer (7) mit Sterilluft derart beaufschlagt wird, daß sie einen Überdruck gegenüber der Außenatmosphäre aufweist.

5. Maschine nach einem der Ansprüche 1 bis 4, **dadurch gekennzeichnet,** daß die Sterilkammer (7) im Bereich einer Übergabestelle der Verschlußkappen (3) an die Flaschen (6) eine Öffnung gegenüber dem Flaschenbefüllraum aufweist, der Flaschenbefüllraum seinerseits als Sterilraum ausgebildet ist, in dem ein gegenüber der Sterilkammer (7) höherer Druck herrscht.

6. Maschine nach einem der Ansprüche 3 bis 5, **dadurch gekennzeichnet,** daß die Aufnahmeglieder (14) für die Verschlußkappen (3) im Sternrad (12) als die Verschlußkappen (3) zumindest bereichsweise umgreifende Klemmhalter (15) ausgebildet sind.

7. Maschine nach Anspruch 6, **dadurch gekennzeichnet,** daß in den Aufnahmegliedern (14) Elastomerglieder als Klemmhalter (15) oder mechanische Glieder angeordnet sind.

8. Maschine nach einem der Ansprüche 3 bis 7, **dadurch gekennzeichnet,** daß dem Sternrad (12) ein Stempel (16) zugeordnet ist, der jeweils die in waagerechte Aufsetzlage (11) gelangte Verschlußkappe (3) aus ihren Aufnahmegliedern (14) herausbewegt und auf eine unter den Aufnahmegliedern (14) befindliche Flasche (6) aufsetzt.

9. Maschine nach Anspruch 8, **dadurch gekennzeichnet,** daß der Stempel (16) eine Saugeinheit (18) oder Greifeinheit zur Halterung der aufzusetzenden Verschlußkappe (3) aufweist.

10. Maschine nach einem der Ansprüche 1 bis 9, **dadurch gekennzeichnet,** daß die Zuführungsbahn (2) für die Verschlußkappen als Rutsche ausgebildet ist, die an einer Einlaßöffnung (19) in die Sterilkammer (7) mündet und an dieser die Verschlußkappen (3) für einen Übergang in die Sterilkammer (7) einzeln mit nach unten weisendem Innenraum (3b) bereitstellt.

11. Maschine nach Anspruch 10, **dadurch gekennzeichnet,** daß der Einlaßöffnung (19) für die Verschlußkappen (3) eine Weitergabevorrichtung (20) zugeordnet ist, die jeweils eine Verschlußkappe (3) aus der Zuführungsbahn (2) entnimmt und der Übergabevorrichtung (5) zuführt.

12. Maschine nach Anspruch 11, **dadurch gekennzeichnet,** daß die Weitergabevorrichtung einen mittels eines Antriebs geradlinig und schwenkbar beweglichen, mit einer Saugvorrichtung oder mechanischer Vorrichtung versehenen Stempel (20) umfaßt.

13. Maschine nach einem der Ansprüche 1 bis 12, **dadurch gekennzeichnet,** daß der Übergabevorrichtung (5) ein Detektor (22) zur Ermittlung der Bestückung der Aufnahmeglieder (14) mit Verschlußkappen (3) zugeordnet ist.

14. Maschine nach einem der Ansprüche 1 bis 13, **dadurch gekennzeichnet,** daß die Sprühvorrichtung (8) einen Ultraschallzerstäuber für eine Feinzerstäubung des Sterilisationsmittels aufweist.

15. Maschine nach Anspruch 14, **dadurch gekennzeichnet,** daß das Sterilisationsmittel im wesentlichen aus Wasserstoffperoxid besteht.

16. Maschine nach einem der Ansprüche 1 bis 15, **dadurch gekennzeichnet,** daß die Sprühvorrichtung (8) der Übergabevorrichtung (5) in einem Bereich zugeordnet ist, in dem die Verschlußkappen (3) eine Lage mit nach oben weisendem Innenraum (3b) einnehmen.

17. Maschine nach einem der Ansprüche 1 bis 16, **dadurch gekennzeichnet,** daß die der Sprühvorrichtung (8) nachgeordnete Trocknungsvorrichtung (9) den Verschlußkappeninnenraum (3b) mit einem das Sterilisationsmittel austreibenden Gas beaufschlagt.

18. Maschine nach Anspruch 17, **dadurch gekennzeichnet,** daß das austreibende Gas aus heißer, sterilisierter Luft besteht.

19. Maschine nach Anspruch 17 oder 18, **dadurch gekennzeichnet,** daß die Trocknungsvorrichtung (9) eine mehrere durchlaufende Verschlußkappen (3) beaufschlagende Trocknungsstrecke bildet.

20. Maschine nach einem der Ansprüche 1 bis 19, **dadurch gekennzeichnet,** daß die Maschine (1) eine Parallelanordnung gleichartiger Bauteile zur Zuführung, Bereitstellung und zum Aufsetzen von Verschlußkappen (3) auf parallel laufende Flaschenreihen umfaßt.

21. Verfahren zum Verschließen von Flaschen mit Verschlußkappen, bei dem die Verschlußkappen entlang einer Transportbahn einer Übergabevorrichtung zugeführt werden, welche die Verschlußkappen einzeln erfaßt und auf Flaschen aufsetzt, **dadurch gekennzeichnet,** daß die Verschlußkappen mit nach unten weisendem Innenraum zugeführt, in eine mit dem Innenraum nach oben weisende Stellung überführt, in dieser mit einem Sterilisationsmittel ausgesprüht, anschließend durch Ausblasen getrocknet und nach Überführen der Verschlußkappen in eine Lage mit nach unten weisendem Innenraum auf die Flaschen aufgesetzt werden.

## Claims

1. A machine (1) for sealing bottles (6), with a conveyor path (2) for feeding the sealing caps (3), an isolating device (4) for offering singled-out sealing caps (3) and a transfer device (5) for placing the singled-out sealing caps (3) on the bottles (6), **characterised in that** at least the transfer device (5) is disposed in a sterile chamber (7) and in that there is associated with the transfer device (5) a spray device (8) for introducing a sterilising agent into the sealing cap interior (3b) and a drying device (5) for expelling sterilising agent from the sealing caps (3).

2. A machine according to claim 1, **characterised in that** the transfer device (5) transfers the sealing caps (3) into a sterilising position (10) with an upwardly directed interior (3b), in which the spray device (8) applies sterilising agent to it, and out of this position into a fitting position (11) in which the interior (3b) is directed downwardly.

3. A machine according to one of claims 1 or 2, **characterised in that** the transfer device (5) comprises, encircling it in a vertical plane, a star wheel (12) which has receiving members (14) regularly distributed around its outer periphery (13) to accommodate the singled-out sealing caps (3).

4. A machine according to one of claims 1 to 3, **characterised in that** the sterile chamber (7) has sterile air applied to it in such a way that it is at a pressure above that of the outside atmosphere.

5. A machine according to one of claims 1 to 4, **characterised in that** in the area of a station in which sealing caps (3) are transferred to the bottles (6), the sterile chamber has an aperture opposite the bottle filling space while for its part the bottle filling space is constructed as a sterile space in which the pressure is higher than that of the sterile chamber (7).

6. A machine according to one of claims 3 to 5, **characterised in that** the members (14) in the star wheel (12) and which are intended to receive sealing caps (3) are constructed as clamping holders (5) which engage around at least portions of the sealing caps (3).

7. A machine according to claim 6, **characterised in that** in the receiving members (14) there are, serving as clamping holders (15), elastomeric members or mechanical members.

8. A machine according to one of claims 3 to 7, **characterised in that** associated with the star wheel (12) is a ram (16) which each time moves the sealing cap (3) which has assumed its horizontal fitting position (11) out of its receiving member (14) and places it on the bottle (6) which is under the receiving member (14).

9. A machine according to claim 8, **characterised in that** the ram (16) has a suction unit (18) or gripper unit for holding the sealing cap (3) which is to be applied.

10. A machine according to one of claims 1 to 9, **characterised in that** the feed path (2) for the sealing caps is constructed as a slide which has an inlet aperture (19) opening into the sterile chamber (7), at which it offers the sealing caps (3) for transfer into the sterile chamber (7) individually with their interior (3b) pointing downwards.

11. A machine according to claim 10, **characterised in that** associated with the inlet aperture (19) for the sealing caps (3) is a passing-on device (20) which takes each time one sealing cap (3) from the feeding path (2) and passes it to the transfer device (5).

12. A machine according to claim 11, **characterised in that** the passing-on device comprises a ram (20) adapted for rectilinear and pivoting movement by means of a drive and with a suction device or mechanical device.

13. A machine according to one of claims 1 to 12, **characterised in that** associated with the transfer device (5) is a detector (22) for ascertaining the loading of the receiving members (14) with sealing caps (3).

14. A machine according to one of claims 1 to 13, **characterised in that** the spray device (8) comprises an ultrasonic atomiser for finely atomising the sterilising agent.

15. A machine according to claim 14, **characterised in that** the sterilising agent consists substantially of hydrogen peroxide.

16. A machine according to one of claims 1 to 15, **characterised in that** the spray device (8) is associated with the transfer device (5) in a region in which the sealing caps (3) assume a position in which their interior (3b) is downwardly directed.

17. A machine according to one of claims I to 16, **characterised in that** the drying device (9) downstream of the spray device (8) subjects to the sealing cap interior (3b) a gas which expels the sterilising agent.

18. A machine according to claim 17, **characterised in that** the expelling gas consists of heated sterilised air.

19. A machine according to claim 17 or 18, **characterised in that** the drying device (9) constitutes a drying path which acts on a plurality of passing sealing caps (3).

20. A machine according to one of claims 1 to 19, **characterised in that** the machine (1) has a parallel arrangement of identical component paths for feeding, offering and fitting sealing caps (3) on parallel passing rows of bottles.

21. A method of sealing bottles with sealing caps in which the sealing caps pass along a transport path and are fed to a transfer device which takes the sealing caps individually and fits them on bottles, **characterised in that** the sealing caps are fed with downwardly directed interiors, are transferred to a position in which the interior is pointing upwards and in which they are sprayed with a sterilising agent and then dried by being blown out and, after the sealing caps have been transferred to a position in which the interior points downwards, they are fitted on the bottles.

## Revendications

1. Machine (1) pour la fermeture de bouteilles (6) par des bouchons (3), avec une bande transporteuse (2) pour l'acheminement des bouchons (3), un dispositif (4) de positionnement un par un des bouchons (3) et un dispositif de transfert (5) jusqu'à la mise en place des bouchons (3) à l'unité sur des bouteilles (6),
**caractérisée en ce que**
dans une chambre stérile (7), est monté, au moins le dispositif de transfert (5), et, au-dessus de ce dispositif de transfert, un dispositif de pulvérisation (8) pour l'arrosage de l'espace intérieur (3b) des bouchons (3) et un dispositif de séchage (9) pour extraire de ces bouchons (3), le produit de stérilisation résiduel.

2. Machine selon la revendication 1,
**caractérisée en ce que**
le dispositif de transfert (5), expose, sur un poste de stérilisation (10) les bouchons (3) avec leur espace intérieur (3b) tourné vers le haut, où le dispositif de stérilisation (8) les arrose d'un produit de stérilisation et les transfère à sa sortie dans une position (11) où leur espace intérieur est retourné vers le bas.

3. Machine selon l'une quelconque des revendication 1 ou 2,
**caractérisée en ce que**
le dispositif de transfert (5) comprend une roue (12) tournant dans un plan vertical, sur la périphérie (13) de laquelle sont répartis, à intervalles réguliers, des éléments récepteurs (14) pour les bouchons (3) mis en file un par un.

4. Machine selon l'une quelconque des revendications 1 à 3,
**caractérisée en ce que**
la chambre stérile (7) contient de l'air stérile en sorte qu'elle se trouve ainsi en surpression par rapport à l'air extérieur.

5. Machine selon l'une quelconque des revendications 1 à 4,
**caractérisée en ce que**
la chambre stérile (7) présente dans la zone de transfert des bouchons (3) au-dessus des bouteilles (6), une ouverture en regard de l'espace de remplissage de ces bouteilles, cet espace de remplissage étant, de son côté, une zone stérile dans laquelle règne une pression supérieure à celle de la chambre stérile (7).

6. Machine selon l'une quelconque des revendication 3 à 5,
**caractérisée en ce que**
les éléments récepteurs (14) placés sur la roue (12) sont formés, au moins en partie, comme des porte-pince (15) entourant les bouchons (3).

7. Machine selon la revendication 6,
**caractérisée en ce que**
dans les éléments récepteurs (14) sont insérés des pièces en élastomère (15) ou des éléments mécaniques.

8. Machine selon l'une quelconque des revendications 3 à 7,
**caractérisée en ce que**
à la roue (12), est affecté un poinçon (16) destiné à extraire les bouchons (3) arrivés dans une position verticale (11), de leurs éléments récepteurs (14), et à les placer au-dessus d'une bouteille (6) située au-dessous de l'élément récepteur (14).

9. Machine selon la revendication 8,
**caractérisée en ce que**
le poinçon (16) comporte un orifice d'aspiration (18) ou un dispositif de prise (18) pour maintenir en place les bouchons (3) avant fermeture.

10. Machine selon l'une quelconque des revendications 1 à 9,
**caractérisée en ce que**
la bande transporteuse (2) des bouchons est constituée par une glissière, qui débouche dans un orifice d'entrée (19) dans la chambre stérile (7) dans laquelle les bouchons (3) sont préparés pour leur introduction individuelle dans la chambre stérile (7), leur espace intérieur (3b) étant dirigé vers le bas.

11. Machine selon la revendication 10,
**caractérisée en ce que**
l'orifice d'introduction (19) des bouchons (3) se prolonge par un dispositif de prolongement (20) destiné à transférer un par un les bouchons (3) depuis la bande transporteuse (2) jusqu'au dispositif de transfert (5).

12. Machine selon la revendication 11,
**caractérisée en ce que**
le dispositif de prolongement (20) comporte un poinçon (20) à déplacements linéaires et rotatifs au moyen d'une commande, et équipé d'un dispositif d'aspiration ou d'un dispositif mécanique.

13. Machine selon l'une quelconque des revendications 1 à 12,
**caractérisée en ce que**
le dispositif de transfert (5) comporte un détecteur (22) pour vérifier la mise en place des bouchons (3) sur les éléments récepteurs (14).

14. Machine selon l'une quelconque des revendications 1 à 13,
**caractérisée en ce que**
le dispositif de pulvérisation (8) comporte un pulvérisateur à ultrasons pour la pulvérisation fine du produit de stérilisation.

15. Machine selon la revendication 14,
**caractérisée en ce que**
le produit de stérilisation est composé pour l'essentiel de peroxyde d'hydrogène.

16. Machine selon l'une quelconque des revendications 1 à 15,
**caractérisée en ce qu**'
au-dessus du dispositif de transfert (5) est placé le dispositif de pulvérisation (8) dans une zone où les bouchons (3) prennent une position dans laquelle leur espace intérieur (3b) se tourne vers le haut.

17. Machine selon l'une quelconque des revendications 1 à 16,
**caractérisée en ce que**
le dispositif de pulvérisation (8) est suivi d'un dispositif de séchage (9) insufflant dans l'espace intérieur (3b) des bouchons (3), un gaz destiné à extraire le produit de stérilisation.

18. Machine selon la revendication 17,
**caractérisée en ce que**
le gaz à extraire est constitué par de l'air chaud stérilisé.

19. Machine selon l'une quelconque des revendications 17 ou 18,
**caractérisée en ce que**
le dispositif de séchage (9) constitue une zone de séchage capable d'agir sur plusieurs bouchons (3) circulant simultanément.

20. Machine selon l'une quelconque des revendications 1 à 19,
**caractérisée en ce que**
la machine (1) comporte un dispositif pour l'acheminement de pièces identiques, la préparation et l'application en parallèle de bouchons (3) sur des rangées parallèles de bouteilles en mouvement.

21. Procédé pour la fermeture de bouteilles par des bouchons, selon lequel les bouchons sont acheminés le long d'une bande transporteuse d'un dispositif de transfert, qui saisit ces bouchons un par un et les applique sur les bouteilles,
**caractérisé en ce que**
les bouchons, présentant leur espace intérieur tourné vers le bas, sont transférés dans une position où leur espace intérieur est tourné vers le haut, où ils reçoivent une pulvérisation de produit de stérilisation, puis sont séchés par insufflation d'air, puis sont transférés dans une position située au-dessus des bouteilles, leur espace intérieur étant retourné vers le bas.
